# EUROPÄISCHE PATENTSCHRIFT

(11) **EP 1 992 965 B1**
(45) Veröffentlichungstag und Bekanntmachung des Hinweises auf die Patenterteilung: **14.06.2017**
(21) Anmeldenummer: 08103379.7
(22) Anmeldetag: 04.04.2008
(51) Int. Cl.: G01T 1/169, A61N 5/10, G01T 1/29

(54) **Verfahren zur Kalibrierung eines Positronen-Emissions-Tomographen einer Strahlentherapievorrichtung sowie Strahlentherapievorrichtung**
Method for calibrating a positron emission tomographer of a radiation therapy device and radiation therapy device
Procédé de calibrage d'un tomographe à émission de positrons d'un dispositif de radiothérapie et dispositif de radiothérapie

(30) Priorität: 02.05.2007 DE 102007020600
(43) Veröffentlichungstag der Anmeldung: 19.11.2008
(73) Patentinhaber: Siemens Healthcare GmbH, 91052 Erlangen (DE)
(72) Erfinder: Rietzel, Eike, 64289, Darmstadt (DE)

(56) Entgegenhaltungen:
- US-A1- 2004 021 065
- Lilli Geworski ET AL: "Multicenter comparison of calibration and cross calibration of PET scanners", Journal of nuclear medicine : official publication, Society of Nuclear Medicine, 1. Mai 2002 (2002-05-01), Seiten 635-639, XP055100498, United States Gefunden im Internet: URL:http://www.ncbi.nlm.nih.gov/pubmed/119 94527
- PAWELKE J ET AL: "In-Beam PET Imaging for the Control of Heavy-Ion Tumour Therapy", IEEE TRANSACTIONS ON NUCLEAR SCIENCE, IEEE SERVICE CENTER, NEW YORK, NY, US, Bd. 44, Nr. 4, 1. August 1997 (1997-08-01) , Seiten 1492-1498, XP011087704, ISSN: 0018-9499, DOI: 10.1109/23.632694

## Beschreibung

Die Erfindung betrifft ein Verfahren zur Kalibrierung eines Positronen-Emissions-Tomographen einer Strahlentherapievorrichtung sowie eine Strahlentherapievorrichtung zur Durchführung eines derartigen Verfahrens, wobei die Strahlentherapievorrichtung insbesondere eine Partikeltherapieanlage ist.

Die Positronen-Emissions-Tomographie (PET) ist ein bildgebendes Verfahren, das vorwiegend in der Nuklearmedizin und in der Strahlentherapie eingesetzt wird. Hierbei wird ein radioaktiver Zerfall, bei dem ein Positron ausgesendet wird, in einem zu untersuchenden Körper nachgewiesen. Das Positron tritt dabei nach kurzer Distanz in Wechselwirkung mit einem Elektron, wobei beide Teilchen vernichtet werden. An deren Stelle entsteht ein Paar von Gammaquanten, die sich in einem Winkel von 180° voneinander entfernen. Diese Gammaquanten durchdringen den zu untersuchenden Körper und werden nach ihrem Austritt von zwei gegenüberliegenden Detektoren registriert. Ein Positronen-Emissions-Tomograph zur Bildgebung umfasst dabei üblicherweise mehrere Gammastrahlung-Detektoren, die üblicherweise nach Art eines Ringes den zu untersuchenden Patienten umschließen. Eine solche Anordnung ist beispielsweise in der DE 32 08 178 A1 beschrieben.

Aus der Veröffentlichung "Multicenter comparison of calibration and cross calibration of PET scanners"; Lilli Geworski et al.; Journal of Nuclear Medicine 2002; 43:635-639, ist eine Studie bekannt, anhand standardisierter Akquisitions- und Rekonstruktionsprotokolle die Kalibrierung und die Kreuzkalibrierung von Positronen-Emissions-Tomographen zu ihren peripheren Geräten im Rahmen einer multizentrischen Studie überprüft werden können. Hierbei wird die Genauigkeit von Scanner-Kalibrierungen getestet und Gründe für mögliche Abweichungen von Referenzstandards zu ermittelt, um die Vergleichbarkeit von an verschiedenen Institutionen erhobenen Patientendaten zu gewährleisten. Die Überprüfung der Kalibrierung und der Kreuzkalibrierung erfolgt hierbei an einer Vielzahl von Positronen-Emissions-Tomographen verschiedener Typen, wobei zur Kalibrierung und zur Kreuzkalibrierung unterschiedliche Phantome, beispielsweise mit einer homogenen und bekannten Aktivitätskonzentration eingesetzt werden.

Pawelke et al. beschreiben in ihrer Veröffentlichung "In-Beam PET Imaging for the control of Heavy-Ion Tumor Therapy, IEEE Transactons on Nuclear Science, Vol. 44, NO. 4, August 1997 ein Verfahren zur in-situ Kontrolle der Tumor-Therapie mit schweren Ionen mittels eines IN-Beam-Positronen-Emissions-Tomographen (PET-Scanner). Die Methode basiert auf der Messung von dynamischen räumlichen Verteilungen von β⁺-Emittern, die durch nuclearen Zerfall während der Bestrahlung und ihrer Beziehung zu der emittierten Dosis erzeugt werden.

In einem Patienten kann der relevante radioaktive Zerfall beispielsweise durch Injektion oder Inhalation eines radioaktiv markierten Radiopharmakons, auch Tracer genannt, induziert werden. Aus der räumlichen Verteilung des Tracers lassen sich beispielsweise Rückschlüsse auf zugrunde liegende Krankheiten gewinnen.

Ein radioaktiver Zerfall, der die Bildung von Positronen beinhaltet, entsteht auch während einer Strahlentherapie durch die Bestrahlung eines Körpers, und zwar abhängig von der applizierten Strahlendosis. PET-Systeme haben sich dabei als ein geeignetes Mittel erwiesen, eine solche Dosisvalidierung bzw. Überwachung der Strahlentherapie und Partikeltherapie durchzuführen. Insbesondere bei der Partikeltherapie werden daher häufig Messungen vorgenommen, um zu prüfen, ob die geplante Strahlendosis mit der tatsächlich applizierten Dosis übereinstimmt und/oder ob die räumliche Verteilung einer applizierten Dosis mit einer gewünschten räumlichen Verteilung übereinstimmt.

PET-Systeme können dabei an die Erfordernisse einer Partikeltherapieanlage angepasst werden und von der herkömmlichen Ringform abweichen. Es sind beispielsweise so genannte In-Beam-PET-Systeme bekannt, die lediglich aus zwei gegenüberliegenden Detektoren bestehen. Die zusätzliche Öffnung zwischen den beiden Detektoren eines In-Beam-PET-Systems erlaubt z.B. eine freiere Positionierung des Patienten oder eine Bestrahlung des Patienten mit einem Strahl durch diese Öffnung, ohne dass der Strahl auf die Detektoren trifft.

Um eine genaue Dosisvalidierung zu ermöglichen, müssen PET-Systeme in gewissen zeitlichen Abständen, beispielsweise täglich, kalibriert werden. Bislang erfolgt eine Kalibrierung unter Verwendung radioaktiver Quellen. Die radioaktiven Quellen werden dabei definiert in einem Behandlungsraum aufgestellt, in dem auch das PET-System angeordnet ist. Die radioaktiven Quellen erzeugen eine definierte Aktivität, die mit dem PET-System gemessen wird. Diese Messungen werden zur Kalibrierung des PET-Systems verwendet. Dies kann beispielsweise auch eine Überprüfung einer bereits bestehenden Kalibrierung des PET-Systems beinhalten.

Eine derartige Kalibrierung ist aufwändig, da in dem Behandlungsraum eigens radioaktive Quellen aufgestellt und anschließend wieder entfernt werden müssen. Dieser Prozess erfordert eine manuelle Intervention, verursacht Kosten und kann fehlerbehaftet sein.

Es ist die Aufgabe der Erfindung, ein Verfahren anzugeben, bei dem mit einfachen Mitteln kostengünstig eine Kalibrierung eines Positronen-Emissions-Tomographen einer Strahlentherapievorrichtung durchgeführt werden kann. Weiterhin ist es die Aufgabe der Erfindung, eine Strahlentherapievorrichtung zur Durchführung eines derartigen Verfahrens bereitzustellen.

Die Aufgabe wird erfindungsgemäß durch ein Verfahren nach Anspruch 1 sowie durch eine Strahlentherapievorrichtung nach Anspruch 8 gelöst. Vorteilhafte Weiterbildungen finden sich in den Merkmalen der abhängigen Ansprüche.

Bei dem erfindungsgemäßen Verfahren zur Kalibrierung eines Positronen-Emissions-Tomographen einer Strahlentherapievorrichtung
- wird zumindest eine definierte Strahlendosis in einem Probenkörper insbesondere an einem vordefinierten Ort in einem Behandlungsraum appliziert,
- wird die durch die Strahlendosis erzeugte Aktivität mit dem Positronen-Emissions-Tomographen gemessen und
- der Positronen-Emissions-Tomograph mithilfe der gemessenen Aktivität kalibriert.

Dem Verfahren liegt die Idee zu Grunde, dass bei einer Strahlentherapievorrichtung eine definierte Strahlendosis mit dem Behandlungsstrahl in einem Probenkörper appliziert werden kann. Dies ist möglich, da bei einer Strahlentherapievorrichtung die Eigenschaften des Behandlungsstrahls hinsichtlich der Genauigkeit der applizierten Dosis und der Genauigkeit des Applikationsortes in separaten Verfahren überprüft werden und daher als qualitätsgesichert vorausgesetzt werden dürfen. Die applizierte Strahlendosis erzeugt in dem Probenkörper eine definierte Radioaktivität, die über den Positronen-Emissions-Tomographen gemessen und zur Kalibrierung verwendet wird.

Die Strahlendosis wird dabei insbesondere an einem definierten Ort im Behandlungsraum, d.h. an einem definierten Ort relativ zu dem Positronen-Emissions-Tomographen, und/oder an einem definierten Ort im Probenkörper appliziert. Dies ist beispielsweise durch eine Steuerung der Ablenkung eines Partikelstrahls bzw. eine Steuerung der Energie des Partikelstrahls in bekannter Weise möglich. Es ist aber beispielsweise auch möglich, die Strahlendosis im Phantom zu applizieren und das Phantom dann anschließend an dem gewünschten Ort im gleichen oder auch in einem anderen Raum zu positionieren.

Mit dem Verfahren ist es folglich möglich, eine Kalibrierung des PET-Systems ohne zusätzliche radioaktive Quellen durchzuführen, wodurch die Automatisierbarkeit des Verfahrens erhöht wird. Insgesamt werden hierdurch Kosten gesenkt und mögliche Fehlerquellen reduziert. Zudem kann das Verfahren flexibler ausgestaltet werden, da die Intensität der Strahlendosis, der Applikationsort und/oder der Applikationszeitpunkt auf einfache Weise flexibel angepasst und geändert werden können, beispielsweise über eine Steuerung des Behandlungsstrahls.

In einer Weiterbildung des Verfahrens
- werden mehrere unterschiedliche Strahlendosen in dem Probenkörper appliziert,
- werden durch die Strahlendosen erzeugten Aktivitäten jeweils mit dem Positronen-Emissions-Tomographen gemessen, und
- wird der Positronen-Emissions-Tomograph mithilfe der gemessenen Aktivitäten kalibriert.

In einer Ausführungsform des Verfahrens unterscheiden
sich die mehreren unterschiedlichen Strahlendosen hinsichtlich ihrer Dosis, ihres Applikationsortes und/oder ihres Applikationszeitpunkts. Auf diese Weise kann eine Kalibrierung genauer durchgeführt werden.

In Ausführungsformen des Verfahrens werden mithilfe der gemessenen Aktivität bzw. der gemessenen Aktivitäten ein Ansprechverhalten von Detektorelementen des Positronen-Emissions-Tomographen und/oder eine Ortsauflösung und/oder eine Ortskodierung des Positronen-Emissions-Tomographen kalibriert.

Beispielsweise eignen sich Strahlendosen, die sich hinsichtlich ihrer Intensität unterscheiden und beispielsweise nacheinander appliziert werden, besonders dafür, das Ansprechverhalten der Detektorelemente zu kalibrieren.

An unterschiedlichen Orten applizierte Strahlendosen eignen sich zur Kalibrierung der Ortsauflösung oder der Ortskodierung des PET-Systems. Beispielsweise können die definierten Strahlendosen entsprechend einem dreidimensionalen, vordefinierten Muster aufgeteilt appliziert werden. Dies erlaubt eine genauere Kalibrierung der Ortsauflösung und/oder der Ortskodierung des PET-Systems, indem die gemessene Aktivität mit dem dreidimensionalen, vordefinierten Muster abgestimmt wird. Aus dem dreidimensionalen Muster lassen sich folglich zusätzliche Informationen über die räumliche Verteilung der induzierten Radioaktivität gewinnen. Diese Information kann zur Kalibrierung des PET-Systems zusätzlich einfließen.

Der verwendete Probenkörper, in dem die Strahlendosis appliziert wird, kann beispielsweise ein Festkörper-Phantom sein, insbesondere ein so genanntes PMMA-Phantom (für Polymethylmethacrylat-Phantom) oder ein anderes bekanntes Phantom.

Insbesondere, wenn die Strahlentherapievorrichtung als Partikeltherapieanlage ausgebildet ist, kann das Verfahren angewendet werden. Als definierte Strahlendosis gilt hierbei eine durch den Partikelstrahl applizierte Partikeldosis.

Die erfindungsgemäße Strahlentherapievorrichtung, die insbesondere als Partikeltherapieanlage ausgebildet ist, weist einen Positronen-Emissions-Tomographen in einem Behandlungsraum auf, wobei die Strahlentherapievorrichtung bzw. der Positronen-Emissions-Tomograph zur Durchführung eines Kalibrierungsverfahrens nach einem der Ansprüche 1 bis 7 ausgebildet ist.

Ausführungsformen der Erfindung sowie vorteilhafte Weiterbildungen gemäß den Merkmalen der abhängigen Ansprüche werden anhand der folgenden Zeichnung näher erläutert, ohne jedoch darauf beschränkt zu sein. Es zeigen:
- Figur 1: einen schematischen Überblick über eine Partikeltherapieanlage,
- Figur 2: eine Frontalansicht eines Positronen-Emissions-Tomographen in einem Behandlungsraum einer Partikeltherapieanlage, in dessen Zentrum ein Probenkörper zur Kalibrierung angeordnet ist,
- Figur 3: eine seitliche Ansicht des Positronen-Emissions-Tomographen mit dem Probenkörper, und
- Figur 4: eine schematische Darstellung der Verfahrensschritte, die zur Kalibrierung eines Positronen-Emissions-Tomographen eingesetzt werden.

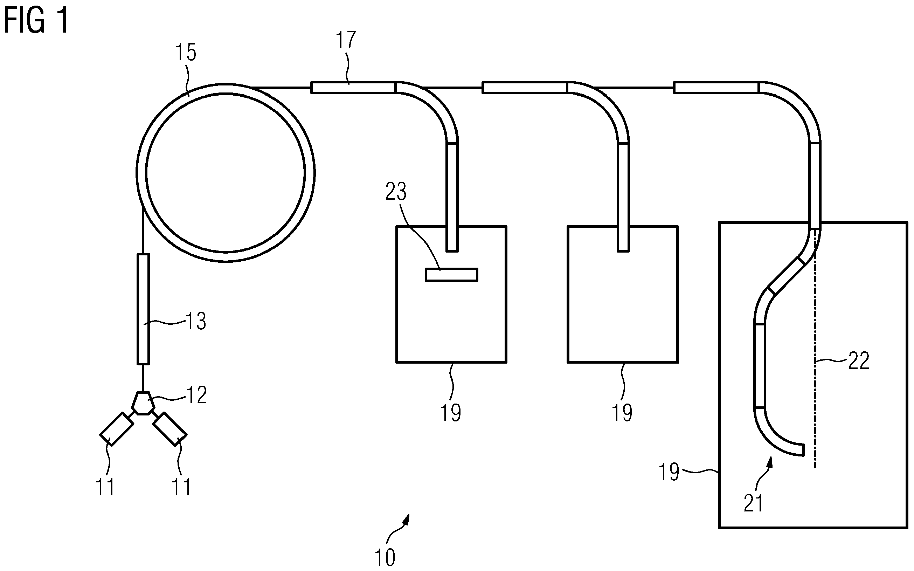

Figur 1 zeigt einen schematischen Überblick über den Aufbau einer Partikeltherapieanlage 10. In einer Partikeltherapieanlage 10 erfolgt eine Bestrahlung eines Körpers, insbesondere eines tumorerkrankten Gewebes, mit einem Partikelstrahl.

Als Partikel werden vornehmlich Ionen wie beispielsweise Protonen, Pionen, Heliumionen, Kohlenstoffionen oder andere Ionensorten eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den Beschleuniger 15 verlassen, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Der anhand der Figur 1 dargestellte Grundaufbau einer Partikeltherapieanlage 10 ist typisch für viele Partikeltherapieanlagen, kann aber auch hiervon abweichen. Die nachfolgend beschriebenen Ausführungsbeispiele sind sowohl in Zusammenhang mit der anhand der Figur 1 dargestellten Partikeltherapieanlage als auch mit anderen Partikeltherapieanlagen oder Strahlentherapieanlagen einsetzbar.

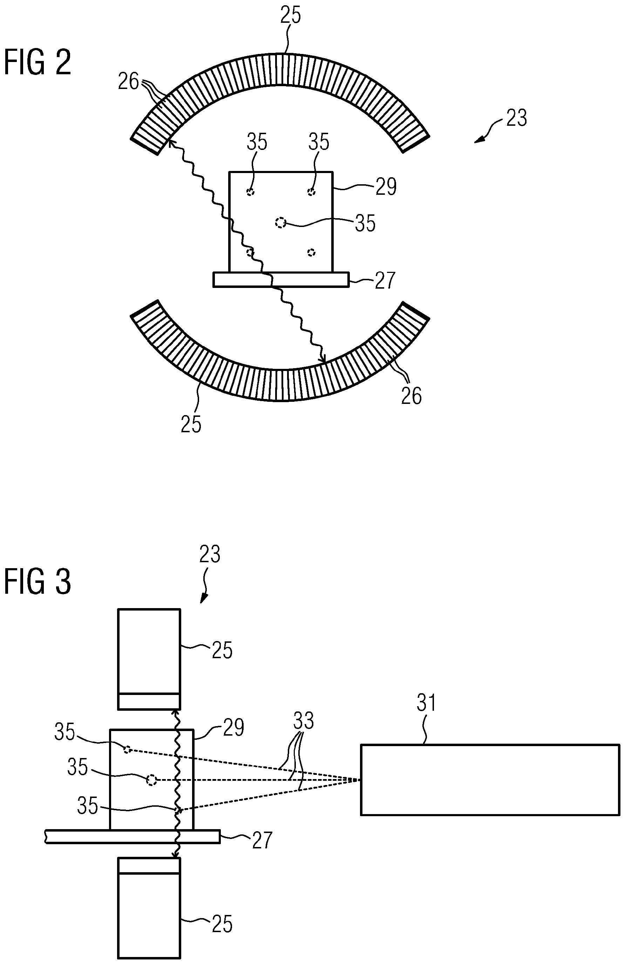

In einem der Bestrahlungsräume 19 ist ein PET-System 23 angedeutet, mit dem eine Dosisvalidierung bzw. eine Überwachung während einer Bestrahlungssitzung im Rahmen der Partikeltherapie durchgeführt werden kann. Eine genauere Darstellung des PET-Systems 23, das mithilfe eines Probenkörpers wie folgt beschrieben kalibriert werden kann, ist in Figur 2 bzw. in Figur 3 gezeigt.

Figur 2 zeigt eine Frontalansicht eines ringförmig ausgebildeten PET-Systems 23. Der Detektorring besteht dabei beispielsweise aus zwei gegenüberliegenden Ringsegmenten 25 an denen jeweils eine Vielzahl von Detektorelementen 26 angeordnet sind. Eine derartige Ausgestaltung des Detektorrings erlaubt eine freiere Positionierbarkeit einer horizontalen Patientenliege, beispielsweise sowohl senkrecht zur Ebene des Detektorrings als auch entlang dieser Ebene hierzu.

Im Zentrum des Detektorrings ist zur Kalibrierung des PET-Systems 23 ein Probenkörper 29 auf einer Tischplatte 27 einer Patientenliege angeordnet, beispielsweise ein quaderförmig ausgebildetes PMMA-Phantom.

Figur 3 zeigt eine seitliche Ansicht des ringförmig ausgebildeten PET-Systems 23 mit dem Probenkörper 29. In Figur 3 ist ebenso ein Strahlauslass 31 sichtbar, aus dem in einem Behandlungs- bzw. Bestrahlungsraum der Partikelstrahl 33 austritt und auf einen oder mehrere verschiedene Bereiche im Probenkörper 29 gerichtet werden kann.

Mit dem Partikelstrahl der Partikeltherapieanlage können nun in dem Probenkörper 29 eine oder - wie hier gezeigt - mehrere in einem räumlichen Muster angeordneten, definierten Partikeldosen 35 appliziert werden. Die Stärke der applizierten Partikeldosen 35 ist in Figur 2 und Figur 3 durch unterschiedlich große Kreise dargestellt. Die einzelnen Partikeldosen 35 werden sukzessive nacheinander in dem Probenkörper appliziert.

Die Steuerung des Partikelstrahls 33 - und damit die Steuerung der applizierten Partikeldosen 35 - wird von einer Steuerungsvorrichtung der Partikeltherapieanlage vorgenommen. Hierzu werden beispielsweise Ablenkmagnete zur Ablenkung des Partikelstrahls 33 entsprechend gesteuert. Zur Steuerung der Eindringtiefe des Partikelstrahls in dem Probenkörper wird seine Energie entsprechend angepasst.

Die applizierten Partikeldosen 35 induzieren im Probenkörper 29 jeweils eine definierte Radioaktivität, die mithilfe des PET-Systems 23 detektiert wird. Da aufgrund der definierten Partikeldosis bzw. Partikeldosen 35 die induzierte Aktivität bekannt ist, kann hierüber eine Kalibrierung des PET-Systems 23 erfolgen.

Die Verwendung eines räumlichen Musters bei der Applikation der Partikeldosen 35 hat den Vorteil, dass die Kalibrierung des PET-Systems 23 genauer als mit einer einzigen Partikeldosis durchgeführt werden kann, da die räumlichen Informationen über das Verteilungsmuster mit den gemessenen Aktivitäten korreliert werden können. Hierdurch kann eine genauere, beispielsweise redundante, Kalibrierung des PET-Systems 23 vorgenommen werden. Andererseits können Partikeldosen 35, die entsprechend einem räumlichen Muster appliziert worden sind, auch dafür verwendet werden, die Ortsauflösung des PET-Systems 23 zu kalibrieren. Hierfür kann eine Abgleichung des räumlichen Musters mit der gemessenen Aktivität des PET-Systems 23 erfolgen.

Nacheinander applizierte, in ihrer Intensität unterschiedliche Partikeldosen 35 können insbesondere zu einer Kalibrierung des Ansprechverhaltens einzelner Detektorelemente 26 verwendet werden.

Auch wenn in Figur 2 und Figur 3 die Kalibrierung für einen Partikelstrahl beschrieben ist, der aktiv gescannt werden kann, kann die Kalibrierung ebenso bei einer passiven Strahlapplikation, d.h. bei einer Strahlapplikation, bei der der Behandlungsstrahl durch passive Elemente im Strahlverlauf geformt wird, eingesetzt werden.

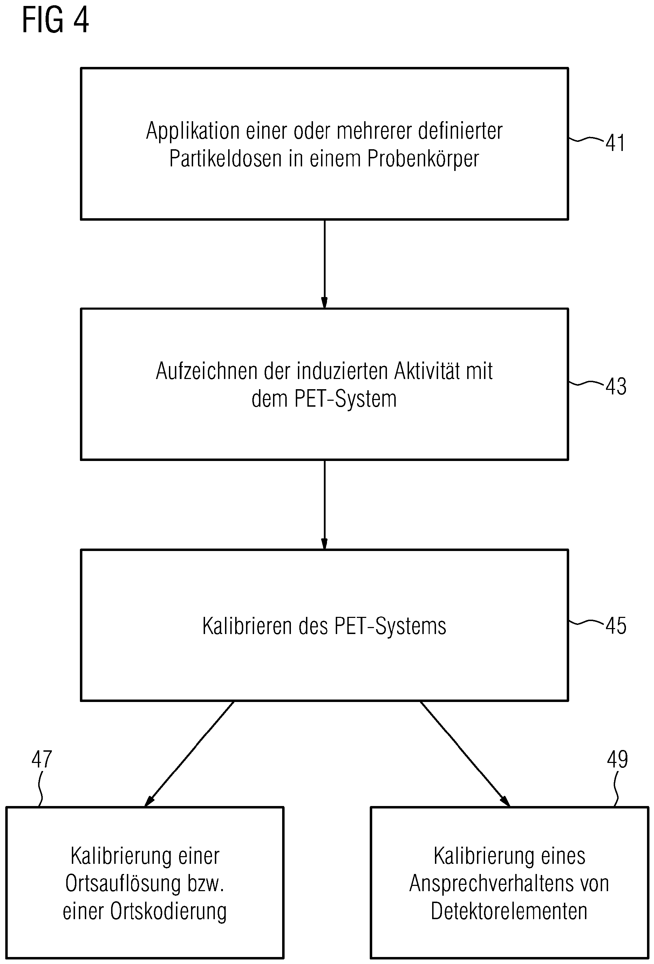

Figur 4 zeigt eine schematische Darstellung der Verfahrensschritte, die zur Kalibrierung eines Positronen-Emissions-Tomographen eingesetzt werden.

In einem ersten Schritt 41 werden in einem relativ zu dem PET-System platzierten Probenkörper eine oder mehrere definierte Partikeldosen mithilfe des Partikelstrahls der Partikeltherapieanlage appliziert. Die applizierte bzw. die applizierten Partikeldosen induzieren im Probenkörper radioaktive Zerfälle, deren Aktivität mithilfe des PET-Systems in einem zweiten Schritt 43 gemessen wird. Anschließend folgt in einem dritten Schritt 45 eine Kalibrierung des PET-Systems mithilfe der gemessenen Radioaktivität. Die Kalibrierung kann dabei beispielsweise anhand der direkt gemessenen Rohdaten erfolgen, zur Kalibrierung kann aber auch eine Rekonstruktion eines PET-Bildes mit den Rohdaten erfolgen, dass dann zur Kalibrierung verwendet wird.

Bei der Kalibrierung kann beispielsweise in einem vierten Schritt 47 eine Ortsauflösung bzw. eine Ortskodierung des PET-Systems kalibriert werden, oder - je nach Ausgestaltung des Verfahrens - in einem fünften Schritt 49 das Ansprechverhalten einzelner Detektorelemente.

Auch wenn in den Figuren das Verfahren im Rahmen einer Partikeltherapie beschrieben worden ist, kann das Verfahren auch bei einer herkömmlichen Strahlentherapie z.B. mit Röntgenstrahlen eingesetzt werden.

## Patentansprüche

1. Verfahren zur Kalibrierung eines Positronen-Emissions-Tomographen (23) einer Strahlentherapievorrichtung (10), indem:
- zumindest eine definierte Strahlendosis (35) in einem Probenkörper (29) appliziert wird,
- die durch die Strahlendosis (35) erzeugte Aktivität mit dem Positronen-Emissions-Tomographen (23) gemessen wird, und
- der Positronen-Emissions-Tomograph (23) mithilfe der gemessenen Aktivität kalibriert wird.

2. Verfahren nach Anspruch 1, wobei
die definierte Strahlendosis (35) an einem definierten Ort relativ zu dem Positronen-Emissions-Tomographen (23) und/oder an einem definierten Ort im Probenkörper (29) appliziert wird.

3. Verfahren nach Anspruch 1 oder 2, wobei
- mehrere unterschiedliche Strahlendosen (35) in dem Probenkörper (29) appliziert werden,
- die durch die Strahlendosen (35) erzeugten Aktivitäten jeweils mit dem Positronen-Emissions-Tomographen (23) gemessen werden,
- der Positronen-Emissions-Tomograph (23) mithilfe der gemessenen Aktivitäten kalibriert wird.

4. Verfahren nach Anspruch 3, wobei
sich die mehreren unterschiedlichen Strahlendosen (35) in ihrer Dosis, in ihrem Applikationsort und/oder in ihrem Applikationszeitpunkt unterscheiden.

5. Verfahren nach einem der Ansprüche 1 bis 4, wobei mithilfe der gemessenen Aktivität ein Ansprechverhalten von Detektorelementen des Positronen-Emissions-Tomographen (23) und/oder eine Ortsauflösung und/oder eine Ortskodierung des Positronen-Emissions-Tomographen (23) kalibriert werden.

6. Verfahren nach einem der Ansprüche 1 bis 5, wobei der Probenkörper (29) ein Polymethylmethacrylat-Phantom ist.

7. Verfahren nach einem der Ansprüche 1 bis 6, wobei die Strahlentherapievorrichtung als Partikeltherapieanlage ausgebildet ist, mit der zumindest eine definierte Partikeldosis als definierte Strahlendosis in dem Probenkörper appliziert wird.

8. Strahlentherapievorrichtung, insbesondere Partikeltherapieanlage, zur Applikation einer definierten Strahlendosis in einem Probenkörper, mit einem Positronen-Emissions-Tomographen (23) in zumindest einem Behandlungsraum (19), wobei die Strahlentherapievorrichtung (10) und der Positronen-Emissions-Tomograph (23) zur Durchführung eines Verfahrens zur Kalibrierung des Positronen-Emissions-Tomographen (23) nach einem der Ansprüche 1 bis 7 ausgebildet sind.

## Claims

1. Method for calibrating a positron emission tomography system (23) in a radiation therapy device (10), by:
- at least one defined radiation dose (35) being applied in a test specimen (29),
- the activity generated by the radiation dose (35) being measured with the positron emission tomography system (23) and
- the positron emission tomography system (23) being calibrated with the aid of the measured activity.

2. Method according to claim 1, wherein
the defined radiation dose (35) is applied at a defined location in relation to the positron emission tomography system (23) and/or at a defined location in the test specimen (29).

3. Method according to claim 1 or 2, wherein
- a number of different radiation doses (35) are applied in the test specimen (29),
- the activities generated by the radiation doses (35) are each measured with the positron emission tomography system (23),
- the positron emission tomography system (23) is calibrated with the aid of the measured activities.

4. Method according to claim 3, wherein
the number of different radiation doses (35) differ in terms of their dose, their application location and/or the time of their application.

5. Method according to one of claims 1 to 4, wherein a response behaviour of detector elements in the positron emission tomography system (23) and/or a spatial resolution and/or a spatial encoding of the positron emission tomography system (23) are calibrated with the aid of the measured activity.

6. Method according to one of claims 1 to 5, wherein the test specimen (29) is a polymethylmethacrylate phantom.

7. Method according to one of claims 1 to 6, wherein the radiation therapy device is embodied as a particle therapy system, with which at least one defined particle dose is applied as a defined radiation dose in the test specimen.

8. Radiation therapy device, in particular particle therapy system, for applying a defined radiation dose in a test specimen, with a positron emission tomography system (23) in at least one treatment room (19), wherein the radiation therapy device (10) and the positron emission tomography system (23) are embodied to carry out a method for calibrating the positron emission tomography system (23) according to one of claims 1 to 7.

## Revendications

1. Procédé d'étalonnage d'un tomographe (23) à émission de positrons d'un dispositif (10) de radiothérapie en :
- appliquant au moins une dose (35) de rayonnement définie à un corps (29) d'échantillon,
- mesurant, par le tomographe (23) à émission de positrons, l'activité produite par la dose (35) de rayonnement et
- étalonnant le tomographe (23) d'émission à positrons à l'aide de l'activité mesurée.

2. Procédé suivant la revendication 1, dans lequel
on applique la dose (35) de rayonnement définie à un emplacement défini par rapport au tomographe (23) à émission de positrons et/ou à un emplacement défini du corps (29) d'échantillon.

3. Procédé suivant la revendication 1 ou 2, dans lequel
- on applique plusieurs doses (35) de rayonnement différentes au corps (29) d'échantillon,
- on mesure, par le tomographe (23) d'émission à positrons, les activités produites par les doses (35) de rayonnement,
- on étalonne le tomographe (23) à émission de positrons à l'aide des activités mesurées.

4. Procédé suivant la revendication 3, dans lequel
les plusieurs doses (35) de rayonnement différentes se distinguent par leur dose, par leur emplacement d'application et/ou par leur instant d'application.

5. Procédé suivant l'une des revendications 1 à 4, dans lequel,
à l'aide de l'activité mesurée, on étalonne un comportement de réponse d'éléments de détecteur du tomographe (23) à émission de positrons et/ou une résolution locale et/ou un codage local du tomographe (23) à émission de positrons.

6. Procédé suivant l'une des revendications 1 à 5, dans lequel le corps (29) d'échantillon est un fantôme en poly (méthacrylate de méthyle).

7. Procédé suivant l'une des revendications 1 à 6, dans lequel le dispositif de radiothérapie est constitué sous la forme d'un dispositif de radiothérapie à particules, 1 par lequel au moins une dose définie de particules est appliquée comme dose de rayonnement définie au corps d'échantillon.

8. Dispositif de radiothérapie, notamment installation de radiothérapie à particules, pour l'application d'une dose de rayonnement définie à un corps d'échantillon,
comprenant un tomographe (23) à émission de positrons dans au moins un espace (19) de traitement, le dispositif (10) de radiothérapie et le tomographe (23) d'émission à positrons étant constitués pour effectuer un procédé d'étalonnage du tomographe (23) à émission de positrons, suivant l'une des revendications 1 à 7.
